(19) Europäisches Patentamt
European Patent Office
Office européen des brevets

(11) EP 4 692 166 A1

(12) **EUROPEAN PATENT APPLICATION**
published in accordance with Art. 153(4) EPC

(43) Date of publication:
**11.02.2026 Bulletin 2026/07**

(21) Application number: **24779721.0**

(22) Date of filing: **19.03.2024**

(51) International Patent Classification (IPC):
***C08G 65/329** (2006.01)*

(52) Cooperative Patent Classification (CPC):
**A61K 47/60; C08G 65/329**

(86) International application number:
**PCT/JP2024/010703**

(87) International publication number:
**WO 2024/203596 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.03.2023 JP 2023051234**

(71) Applicant: **NOF Corporation**
**Shibuya-ku**
**Tokyo 150-6019 (JP)**

(72) Inventors:
- **TACHIYA, Naoto**
  **Kawasaki-shi, Kanagawa 210-0865 (JP)**
- **FUSE, Takumi**
  **Kawasaki-shi, Kanagawa 210-0865 (JP)**
- **SATO, Atsushi**
  **Kawasaki-shi, Kanagawa 210-0865 (JP)**
- **HYODO, Ryo**
  **Toyama-shi, Toyama 930-0806 (JP)**
- **DOI, Yasuhiro**
  **Toyama-shi, Toyama 930-0806 (JP)**

(74) Representative: **Hoffmann Eitle**
**Patent- und Rechtsanwälte PartmbB**
**Arabellastraße 30**
**81925 München (DE)**

# (54) METHOD FOR PRODUCING ACTIVATED POLYETHYLENE GLYCOL DERIVATIVE

(57) To provide a method for producing a polyethylene glycol derivative that has a high activation purity, that can prevent an increase in polydispersity due to an increase in thermal history, and that is industrially feasible.

A method for producing an activated polyethylene glycol derivative having, at a terminal, a functional group reactive with a bio-related substance or a precursor of the functional group includes the following step (A), step (B), and step (C).

Step (A): a step of drying a particulate material of a polyethylene glycol compound having, at a terminal, one or more groups selected from the group consisting of a carboxy group, a mercapto group, a hydroxy group, and an amino group under an atmosphere of 0°C or higher and 50°C or lower to obtain a dried particulate material having a moisture value of 0.10 mass% or less

Step (B): a step of adding an organic solvent containing no more than 200 ppm of water to the dried particulate material obtained in the step (A) to dissolve the dried particulate material in the organic solvent to obtain a solution

Step (C): a step of reacting the polyethylene glycol compound contained in the solution obtained in the step (B) with an activator to obtain an activated polyethylene glycol derivative

FIG. 1

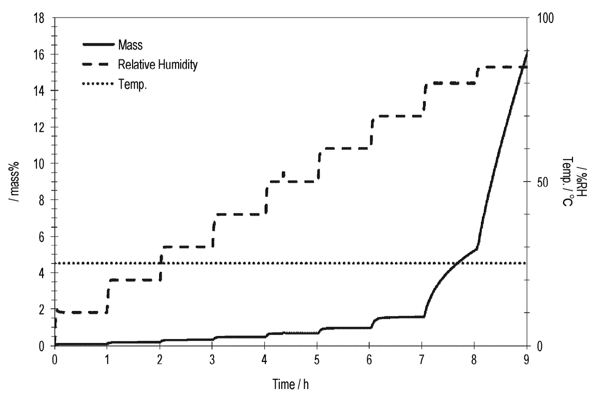


EP 4 692 166 A1

## Description

TECHNICAL FIELD

The present invention relates to a method for producing an activated polyethylene glycol derivative.

BACKGROUND ART

When pharmaceuticals composed of bio-related substances such as hormones, cytokines, and enzymes, are administered into a living organism, they are generally excreted promptly through glomerular filtration in the kidneys or taken up by macrophages in the liver or spleen. Therefore, their half-life in the blood is short, making it difficult to achieve sufficient pharmacological effects. In order to solve this problem, attempts have been made to chemically modify a bio-related substance with a water-soluble polymer such as a polyethylene glycol (hereinafter sometimes referred to as "PEG"). As a result, it is possible to extend the half-life in the blood of the bio-related substances by increasing the molecular weight and forming a hydration layer. It is also well known that these modifications can produce effects such as reduction in toxicity and antigenicity of the bio-related substances, improvement in aggregation properties, and the like.

An activated polyethylene glycol derivative generally has, at a polyethylene glycol chain terminal, an active group that chemically bonds to a functional group such as an amino group, a mercapto group, a carboxy group, or an unsaturated bond present on the surface of a protein to be modified. For example, in the case of modifying an amino group, there is an active group such as a formyl group, an epoxy group, a p-nitrophenyl ester group, or an N-hydroxysuccinimidyl ester group at the polyethylene glycol chain terminal. In the case of modifying a mercapto group, there is an active group such as a mercapto group, a maleimidyl group, a substituted maleimidyl group, an allyl group, or an N-hydroxysuccinimidyl ester group at the polyethylene glycol chain terminal. In the case of modifying a carboxy group, there is an active group such as a mercapto group or an amino group at the polyethylene glycol chain terminal. In the case of modifying an unsaturated bond, there is an active group such as a mercapto group at the polyethylene glycol chain terminal.

As the activated polyethylene glycol derivative used for modification of a drug, it is necessary to use a high-purity product having a small polydispersity (weight average molecular weight (Mw)/number average molecular weight (Mn)) in order to prevent a variation in circulation in blood, immunogenicity, drug efficacy, or the like of the drug from the viewpoint of uniformity of the drug. **In** addition, a conversion rate to a reactive functional group during synthesis of the activated polyethylene glycol derivative is preferably high in order to prevent by-production of PEG-based impurities, PEGylated preparations having different bonds, and the like.

An activated polyethylene glycol derivative having, at a terminal, a functional group reactive with a bio-related substance or a precursor thereof is obtained by reacting a polyethylene glycol compound raw material having a hydroxy group, an amino group, or the like at a terminal with an activator, which is a low molecular weight compound.

However, some activators have hydrolyzability and react sensitively with water in the reaction system, resulting in deactivation. When the activator is deactivated, a purity of the activated polyethylene glycol derivative decreases, and when an excessive amount of the activator is added in anticipation of deactivation, the amount of impurities derived from the activator also increases, which leads to an increase in load of a purification step, so that the amount of the activator needs to be minimized.

A polyethylene glycol compound having, at a terminal, a carboxy group, a mercapto group, a hydroxy group, an amino group, or the like has various properties such as being in the form of a hard wax, a flake obtained by coarsely crushing the hard wax, and a particulate material. A polyethylene glycol compound having a hydroxy group at a terminal is obtained by anionic polymerization of an active hydrogen compound and ethylene oxide. The polymerization is almost under anhydrous conditions, but the compound absorbs moisture when it is taken out in a liquid form from a high-temperature kettle after the polymerization and cooled and solidified in an atmospheric environment. In addition, the polyethylene glycol compound after some reaction or purification may be formed into a particulate material by reprecipitation or the like using a solvent. The particulate material has a surface area larger than that of a wax-like or flake-like solid, and thus has high hygroscopicity, and even when the particulate material is dried during production, a moisture content is likely to increase when the particulate material is handled in an atmospheric environment.

For the above reasons, a particulate material of the polyethylene glycol compound before an activation reaction usually contains 0.1 mass% to 2 mass% of moisture. Since this is a level that influences deactivation of the activator in the activation reaction, it is necessary to reduce the moisture amount in the system by performing some drying step before the reaction.

Patent Literature 1 describes mixing a polyethylene glycol compound with a solvent capable of forming an azeotropic mixture with water to obtain a mixture, then drying the mixture by azeotropic distillation, and then activating the polyethylene glycol compound in the solution.

PRIOR ART DOCUMENT

PATENT LITERATURE

Patent Literature 1: JP2013-227543A

## SUMMARY OF INVENTION

### OBJECT TO BE ACHIEVED BY THE INVENTION

However, as described in Patent Literature 1, in a method for producing an activated polyethylene glycol derivative in which the polyethylene glycol compound in the solution is dried and then activated by azeotropic distillation, there is a possibility that a quality of the activated polyethylene glycol derivative decreases. This is because a thermal history increases by continuously heating the solution of the polyethylene glycol compound as a raw material at a high temperature, and the polydispersity increases due to oxidative decomposition of the PEG chain. In general, as the production scale is larger, the time required for heating and cooling is longer, so that an increase in polydispersity due to an increase in thermal history appears more remarkably.

As described above, although the activated polyethylene glycol derivative is an important material in pharmaceutical applications, it is difficult to easily obtain the activated polyethylene glycol derivative.

An object of the present invention is to provide a method for producing an activated polyethylene glycol derivative that has a high activation purity, that can prevent an increase in polydispersity due to an increase in thermal history, and that is industrially feasible.

### MEANS FOR ACHIEVING THE OBJECT

As a result of intensive studies to achieve the above object, the present inventors have found that by using a polyethylene glycol compound having specific properties as a raw material and performing an activation reaction after performing a specific drying step, an activated polyethylene glycol derivative having a high activation purity and a prevented decrease in quality can be obtained.

That is, the present invention is as follows.

[1] A method for producing an activated polyethylene glycol derivative having, at a terminal, a functional group reactive with a bio-related substance or a precursor of the functional group, the method including the following step (A), step (B), and step (C):

step (A): a step of drying a particulate material of a polyethylene glycol compound having, at a terminal, one or more groups selected from the group consisting of a carboxy group, a mercapto group, a hydroxy group, and an amino group under an atmosphere of 0°C or higher and 50°C or lower to obtain a dried particulate material having a moisture value of 0.10 mass% or less;
step (B): a step of adding an organic solvent containing no more than 200 ppm of water to the dried particulate material obtained in the step (A) to dissolve the dried particulate material in the organic solvent under a sealed environment to obtain a solution; and
step (C): a step of reacting the polyethylene glycol compound contained in the solution obtained in the step (B) with an activator to obtain an activated polyethylene glycol derivative.

[2] The method for producing an activated polyethylene glycol derivative according to [1], in which the organic solvent is an aprotic solvent.
[3] The method for producing an activated polyethylene glycol derivative according to [1] or [2], in which the particulate material in the step (A) has an average particle diameter of 0.1 $\mu$m or more and 10 mm or less.
[4] The method for producing an activated polyethylene glycol derivative according to [1] or [2], in which the polyethylene glycol compound in the step (A) has a number average molecular weight of 2,000 Daltons or more and 80,000 Daltons or less.
[5] The method for producing a polyethylene glycol derivative according to [1] or [2], in which the activated polyethylene glycol derivative is represented by the following formula (1):

PEG-X ⋯ (1)

(in the formula (1),

PEG represents a polyethylene glycol moiety having a linear structure or a branched structure, and

X represents the functional group reactive with the bio-related substance or the precursor of the functional group).

[6] The method for producing a polyethylene glycol derivative according to [1] or [2], in which the activator is one or more compounds selected from the group consisting of disuccinimidyl carbonate, p-nitrophenyl chloroformate, di(1-benzotriazolyl) carbonate, trichlorophenyl chloroformate, p-nitrophenyl succinimidyl carbonate, p-nitrophenyl 1-benzotriazolyl carbonate, pentafluorophenyl chloroformate, 1,1'-carbonyldiimidazole, succinic anhydride, glutaric anhydride, halogenated methanesulfonyl, halogenated trifluoromethanesulfonyl, halogenated p-toluenesulfonyl, an alkyl 6-halogenated hexanoate, an azodicarboxylate, N,N'-dicyclohexylcarbodiimide, and N-hydroxysuccinimide.

EFFECTS OF INVENTION

According to the present invention, it is possible to obtain an effect of increasing the activation purity, preventing an increase in polydispersity due to an increase in thermal history, and producing an industrially feasible activated polyethylene glycol derivative.

BRIEF DESCRIPTION OF DRAWINGS

Fig. 1 is a graph showing a relationship between a temperature, a relative humidity, and a mass change rate of a measurement sample during moisture adsorption/desorption measurement in Experimental Example 1.

EMBODIMENTS FOR CARRYING OUT THE INVENTION

[Activated Polyethylene Glycol Derivative]

An activated polyethylene glycol derivative according to the present invention has, at a terminal, a functional group reactive with a bio-related substance or a precursor of the functional group.
In a preferred embodiment, the activated polyethylene glycol derivative is represented by the following formula (2).

PEG-X ··· (2)

In the formula (2), PEG represents a polyethylene glycol moiety having a linear structure or a branched structure, and X represents the functional group reactive with the bio-related substance or the precursor of the functional group.
More specifically, an activated polyethylene glycol derivative represented by a formula (3) can be exemplified.

[Chem. 1]

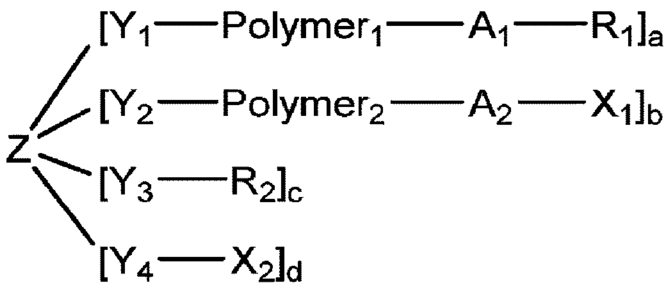

(3)

Here, Z is a residue obtained by removing an active hydrogen group (-GH) from a compound having 2 to 8 active hydrogen groups (Z(GH)n, n = 2 to 8). The active hydrogen group is a functional group having active hydrogen. Examples of the active hydrogen group (-GH) include a hydroxy group, a carboxy group, an amino group, a secondary amino group, and a mercapto group. In the case where the active hydrogen group (-GH) is a hydroxy group or a carboxy group, the residue Z is a dehydroxylated residue, and in the case where the active hydrogen group (-GH) is an amino group, a secondary amino group, or a mercapto group, the residue Z is a dehydrogenated residue.
Specific examples of the compound having 2 to 8 active hydrogen groups (-GH) (Z(GH)n, n = 2 to 8) include: polyhydric alcohols such as ethylene glycol, propylene glycol, trimethylene glycol, isopropylene glycol, butylene glycol, tetramethylene glycol, trimethylolpropane, glycerin, diglycerin, triglycerin, hexaglycerin, pentaerythritol, dipentaerythritol, and xylitol; amino acids or peptides having an amino group, a carboxy group, or a mercapto group such as lysine and

glutamic acid; or compounds such as an organic amine and an organic carboxylic acid.

a, b, c, and d are integers satisfying "$0 \leq a \leq 8$, $0 \leq b < 8$, $0 \leq c \leq 7$, $0 \leq d \leq 7$" and " $1 \leq a+b \leq 8$, $2 \leq a+b+c+d \leq 8$".

$Y_1$, $Y_2$, $Y_3$, and $Y_4$ each independently represent an ether bond, an amide bond, an ester bond, a urethane bond, a carbonate bond, a secondary amino group, a thioether bond, a disulfide bond, a thioester bond, or an alkylene group optionally containing any of these. Preferred examples of the alkylene group include a methylene group, an ethylene group, a propylene group, an isopropylene group, a butylene group, an isobutylene group, a pentylene group, an isopentylene group, and a hexylene group, which may be branched.

Polymer1 and Polymer2 are linear or branched polyethylene glycol chains. The branched polyethylene glycol chain is a polyethylene glycol chain branched into two or more chains via a linker in the middle, and may have a plurality of branching points. An example is a polyethylene glycol chain having two or more branched chains and having a polyhydric alcohol such as glycerin as a branching point, as shown in the following formula (i).

[Chem. 2]

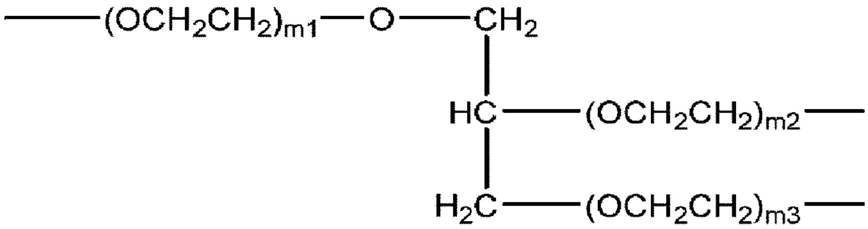

・・・(i)

(Here, m1, m2, and m3 are integers, preferably 1 to 3636, more preferably 1 to 2728, and still more preferably 1 to 1818.)

The PEG in the present invention is a polymer having a polydispersity of preferably 1.2 or less, more preferably 1.1 or less, and most preferably 1.07 or less, as measured by analysis using gel permeation chromatography (GPC).

A number average molecular weight of a PEG moiety in the formula (2) is a value measured by analysis using GPC, and is a number average molecular weight calculated based on a calibration curve prepared using PEGs having various known molecular weights. The number average molecular weight of the PEG moiety is not particularly limited, and is preferably 2,000 Daltons or more and 80,000 Daltons or less, more preferably 2,000 Daltons or more and 60,000 Daltons or less, and still more preferably 2,000 Daltons or more and 50,000 Daltons or less.

In the formula (3), $A_1$ and $A_2$ each independently represent $-L_1-(CH_2)l_1-$, $-L_1-(CH_2)l_1-L_2-(CH_2)l_2$ or a single bond, $L_1$ represents an ether bond, an amide bond, a urethane bond, a secondary amino group, or a single bond, $L_2$ represents an ether bond, an amide bond, a urethane bond, a single bond, or a thioether bond, and $l_1$ and $l_2$ each independently represent an integer of 0 to 5.

$X_1$ and $X_2$ are each independently a functional group reactive with a bio-related substance or a precursor of the functional group. The number of kinds of the functional groups or precursors of the functional groups in the activated polyethylene glycol derivative is not limited to one, and may be two or more.

$R_1$ and $R_2$ are each independently a structure that does not react with the activator in the reaction in the present invention. The structure is not particularly limited, and may have a functional group reactive with a bio-related substance, a precursor of the functional group, or a hydrocarbon. The structure is exemplified below.

An alkynyl group, a substituted alkynyl group (for example, an alkynyl group substituted with a hydrocarbon group having 1 to 5 carbon atoms), an alkenyl group (for example, an allyl group or a vinyl group), an alkyl group, an ester group, a carbonate group (for example, a succinimidyl carbonate group or a p-nitrophenyl carbonate group), a formyl group, an isocyanate group, an isothiocyanate group, an epoxide group, a carboxy group, a maleimidyl group, a substituted maleimidyl group, a dithiopyridyl group, a substituted sulfonyl group (for example, a mesyl group or a tosyl group), a vinylsulfonyl group, an iodoacetamide group, an alkylcarbonyl group, an azide group, an acryloyl group, a sulfonyloxy group (for example, an alkylsulfonyloxy group), and an $\alpha$-haloacetyl group

In the present invention, examples of the functional group reactive with the bio-related substance contained in the activated polyethylene glycol derivative or the precursor of the functional group are given below.

An epoxy group, a p-nitrophenyl ester group, an N-hydroxysuccinimidyl ester group, a mercapto group, a maleimidyl group, a substituted maleimidyl group, an allyl group, an azide group, a biotin group, a formyl group, an amino group, a mesyl group, a tosyl group, a phthalimidyl group, a carboxy group, an acetal-protected formyl group, a 9-fluorenylmethyloxycarbonyl group-protected amino group, a t-butoxycarbonyl group-protected amino group, and a benzyloxycarbonyl group-protected amino group

A production method according to the present invention includes a step (A), a step (B), and a step (C). Hereinafter, each step will be described in order.

Step (A): a step of drying a particulate material of a polyethylene glycol compound having, at a terminal, one or

more groups selected from the group consisting of a carboxy group, a mercapto group, a hydroxy group, and an amino group under an atmosphere of 0°C or higher and 50°C or lower to obtain a dried particulate material having a moisture value of 0.10 mass% or less

The raw material in the step (A) is a polyethylene glycol compound having, at a terminal, one or more selected from the group consisting of a carboxy group, a mercapto group, a hydroxy group, and an amino group. The polyethylene glycol compound is preferably a PEG having a structure represented by a formula (4).

[Chem. 3]

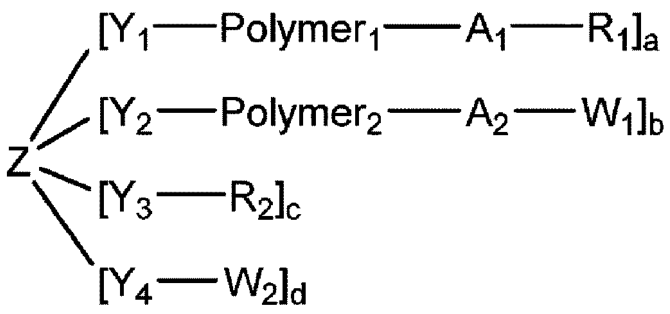

(4)

In the formula (4), $W_1$ and $W_2$ each independently represent a carboxy group, a mercapto group, a hydroxy group, and an amino group. The other symbols have the same definitions as in the formula (2).

The polyethylene glycol compound is in the form of a dry particulate material. The drying method is not particularly limited, and examples thereof include drying under a reduced pressure and drying using dry gas circulation. The particulate material has an average particle diameter of preferably 0.1 μm or more and 10 mm or less, and more preferably 0.1 μm or more and 1 mm or less, as a value measured by analysis using a sieving method.

A container used in the method according to the present invention is one in which the particulate material of the polyethylene glycol compound can be dried and a solvent can be added to the dried particulate material of the polyethylene glycol derivative without contact with the atmosphere. Here, "without contact with the atmosphere" means that the contact with the atmosphere is prevented by making the container in a sealed state or filling the container with an inert gas to create a positive pressure in the container with the inert gas, or by accommodating the container in an apparatus in a sealed state and filling the apparatus with the inert gas or filling the apparatus with an inert gas to create a positive pressure in the apparatus with the inert gas.

The moisture value of the dried particulate material is preferably 0.10 mass% or less.

When the particulate material is dried by drying under a reduced pressure, the pressure is preferably -0.01 MPaG or less and -1.0 MPaG or more, and more preferably -0.1 MPaG or less and -1.0 MPaG or more.

The atmosphere during the drying under a reduced pressure is not particularly limited, and is preferably an inert gas such as a nitrogen or argon gas.

When the particulate material is dried using dry gas circulation, the type of the gas is not particularly limited, and is preferably an inert gas such as a nitrogen or argon gas.

The temperature during drying the particulate material is not particularly limited, and any temperature equal to or lower than a melting point of the particulate material of the polyethylene glycol compound can be applied. The temperature is preferably 0°C or higher and 50°C or lower, and more preferably 15°C or higher and 35°C or lower.

Step (B): a step of adding an organic solvent containing no more than 200 ppm of water to the dried particulate material obtained in the step (A) to dissolve the dried particulate material in the organic solvent to obtain a solution

When an organic solvent containing no more than 200 ppm of water is added to the dried particulate material and the dried particulate material is dissolved to obtain a solution, a method of adding the organic solvent is not particularly limited. The organic solvent may be added into the container without contact of the particulate material of the polyethylene glycol derivative with the outside air. Examples of the method of adding the organic solvent include pump transfer, pressurization transfer, and depressurization transfer.

The mass of water of the organic solvent used in the steps (B) and (C) is 200 ppm or less, preferably 150 ppm or less, and more preferably 100 ppm or less. The lower limit of the mass of water is not particularly limited and may be 0 ppm.

The organic solvent used in the present invention is preferably an aprotic solvent that does not react with the activator. Examples of the solvent include toluene, benzene, ethyl acetate, dimethylformamide, dimethylsulfoxide, diethyl ether, methyl tert-butyl ether, tetrahydrofuran, acetonitrile, acetone, chloroform, and dichloromethane. The necessary conditions of the solvent in the present invention are conditions in which the PEG is dissolved, the reaction proceeds, the activator is not deactivated, and the functional group after the reaction is not decomposed. A lower alcohol such as

methanol or ethanol, which is generally used as a reaction solvent, is not preferred from the viewpoint that the activator or the functional group after the reaction is highly likely to be decomposed.

Step (C): a step of reacting the polyethylene glycol compound contained in the solution obtained in the step (B) with an activator to obtain an activated polyethylene glycol derivative

The activator reacts with one or more groups selected from the group consisting of a carboxy group, a mercapto group, a hydroxy group, and an amino group in a polyethylene glycol compound to provide an activated polyethylene glycol derivative having, at a terminal, a functional group reactive with a bio-related substance or a precursor of the functional group.

Preferred examples of the activator include the following.

One or more compounds selected from the group consisting of disuccinimidyl carbonate, p-nitrophenyl chloroformate, di(1-benzotriazolyl) carbonate, trichlorophenyl chloroformate, p-nitrophenyl succinimidyl carbonate, p-nitrophenyl 1-benzotriazolyl carbonate, pentafluorophenyl chloroformate, 1,1'-carbonyldiimidazole, succinic anhydride, glutaric anhydride, halogenated methanesulfonyl, halogenated trifluoromethanesulfonyl, halogenated p-toluenesulfonyl, an alkyl 6-halogenated hexanoate, an azodicarboxylate, N,N'-dicyclohexylcarbodiimide, and N-hydroxysuccinimide

In the reaction in the step (C), a catalyst such as a base may be used as a reaction accelerator, and the base may be either an inorganic base or an organic base.

Examples of the base include salts or amine compounds such as hydroxides, carbonates, bicarbonates, acetates, citrates, phosphates, borates, citrates, phthalates, tartrates, or lactates of alkali metals and alkaline earth metals. Exemplary salts include sodium carbonate, sodium hydrogen carbonate, and sodium acetate. As the amine compound, a primary amine and a secondary amine may react with the activator or the functional group after the reaction, and thus a tertiary amine is more preferred. Examples of exemplary bases as the tertiary amine include triethylamine, N-methylmorpholine, N-phenylmorpholine, N,N-diisopropylethylamine, pyridine, 2,6-lutidine, 4-dimethylaminopyridine, 1,4-diazabicyclo[2.2.2]octane, and diazabicycloundecene. In the case of using salts, since the salts may contain moisture, the salts may be added together with the particulate material of the polyethylene glycol compound in the step (A) and dried.

## EXAMPLES

Hereinafter, the present invention will be described in more detail with reference to Examples.

A GPC polydispersity of the polyethylene glycol derivative was measured by the following analysis method A. An activation rate of the polyethylene glycol derivative was measured by the following analysis method B. A moisture value of the polyethylene glycol derivative was measured by the following analysis method C.

<Analysis Method for GPC Polydispersity>

(Analysis Method A)

GPC system: Prominence manufactured by Shimadzu Corporation
Detector: differential refractometer RID-20A manufactured by Shimadzu Corporation
GPC column: two PL gel MIXED-D manufactured by Agilent Technologies connected in series
Column oven temperature: 65°C
Eluent: N,N-dimethylformamide containing 10 mmol/L lithium bromide
Flow rate: 0.7 mL/min
Sample concentration: 1 mg/mL
Extraction capacity: 0.1 mL
Standard product for calibration curve: Polymer Standards for GPC of PEG having a molecular weight of 600 to 70,000, manufactured by Agilent Technologies
Data analysis: Lab Solution manufactured by Shimadzu Corporation

<Method for Analyzing Activation Rate>

The activation rate was analyzed by proton nuclear magnetic resonance (1H-NMR).

(Analysis Method B)

1H-NMR apparatus: JNM-ECA600 manufactured by JEOL
Measurement temperature: 25°C
Measurement solvent: deuterated chloroform manufactured by KANTO KAGAKU.
Sample concentration: 25 mg/mL
Measurement nuclide: 1H
Number of times of integration: 64 times to 256 times
Data analysis: ALICE2 manufactured by JEOL

<Method for Analyzing Moisture Value>

The moisture value was measured with a Karl Fischer moisture meter.

(Analysis Method C)

Karl Fischer moisture meter: MKC-501 manufactured by KYOTO ELECTRONICS MANUFACTURING CO., LTD.
Karl Fischer reagent: HYDRANAL COULOMAT AG (anode) manufactured by Honeywell, HYDRANAL COULOMAT CG (cathode) manufactured by Honeywell
Measurement temperature: 25°C
Measurement amount: 1 g

<Experimental Example 1>

In order to check the hygroscopicity of the particulate material of the polyethylene glycol compound, the following studies were performed.

A particulate material of a raw material (polyethylene glycol compound) represented by a formula (5) (Mw: 2,000 Daltons, reactive functional group: one hydroxy group, 27.8 mg) was subjected to moisture adsorption/desorption measurement using IGA sorp manufactured by Hiden Isochema. As a pretreatment, the particulate material of the polyethylene glycol compound was placed in a SUS mesh holder and dried at 25°C and a relative humidity of 0% RH for 5 hours to obtain a dried particulate material.

The dried particulate material (27.46 mg) was allowed to stand for 1 hour in an environment controlled to any relative humidity at 25°C, and the mass after standing was measured. The measurement results are shown in Table 1 and Fig. 1.

As a result, it is revealed that, as the relative humidity increases, the dried particulate material of the polyethylene glycol compound absorbs moisture and the mass increases. In addition, it is found that the particulate material of the polyethylene glycol compound absorbs moisture immediately after the increase in relative humidity and is strongly influenced by the environmental temperature and humidity.

Therefore, in the case where the particulate material of the polyethylene glycol compound is reacted with an activator in order to obtain a target activated polyethylene glycol derivative, there is a high possibility that the progress of the reaction is hindered when the particulate material of the polyethylene glycol compound having absorbed moisture is used as a raw material.

[Chem. 4]

$$H-(OCH_2CH_2)_n-OCH_3 \qquad (5)$$

(n = about 45)

**[Table 1]**

| Relative humidity (%RH) | Mass change rate (mass%) |
|---|---|
| (Initial) | 0 |
| 10 | 0.08 |
| 20 | 0.18 |

(continued)

| Relative humidity (%RH) | Mass change rate (mass%) |
|---|---|
| 30 | 0.31 |
| 40 | 0.47 |
| 50 | 0.67 |
| 60 | 0.96 |
| 70 | 1.56 |
| 80 | 5.27 |
| 85 | 16.38 |

<Experimental Example 2>

In order to check the quality change of the polyethylene glycol compound in the case where azeotropic distillation was performed to dry the particulate material of the polyethylene glycol compound, the following studies were performed.

Into a 300 mL four-necked round-bottom flask equipped with a mechanical stirrer, a Dimroth cooling tube, a moisture meter receiver, a thermometer, and a nitrogen injection tube were charged a particulate material of a polyethylene glycol compound represented by a formula (6) (Mw: 40,000 Daltons, reactive functional group: two hydroxy groups, 10 g, moisture value: 1.1 mass%) and toluene (70 g, moisture value: 68 ppm), and the mixture was dissolved at 50°C with a mantle heater while performing stirring under nitrogen. Thereafter, azeotropic distillation was performed at 115°C using a mantle heater. Table 2 shows a relationship between a azeotropic distillation time and the GPC polydispersity (Mw/Mn) in the analysis method A.

As a result, it is revealed that, with the extension of the azeotropic distillation time, the polyethylene glycol compound deteriorates and the polydispersity is increased. It is found that, in the case of drying by azeotropic distillation, as the production scale increases, the heating time until the temperature reaches a temperature at which azeotropic distillation can be performed is extended, so that deterioration of the polyethylene glycol compound is promoted and the polydispersity is increased.

[Chem. 5]

$$H - (OCH_2CH_2)_n - OH \qquad \text{Formula (6)}$$

(n = about 909)

[Table 2]

| Azeotropic distillation time (hours) | GPC polydispersity (Mw/Mn) |
|---|---|
| (Initial) | 1.11 |
| 1 | 1.11 |
| 3 | 1.12 |
| 6 | 1.13 |
| 9 | 1.15 |
| 12 | 1.16 |
| 18 | 1.19 |
| 24 | 1.25 |

<Example 1>

Into a 300 mL four-necked round-bottom flask equipped with a mechanical stirrer, an L-shaped tube connected to

a vacuum pump, a check valve, a three-way cock connected to a nitrogen line, and a glass stopper was charged a particulate material of a polyethylene glycol compound represented by the formula (6) (Mw: 40,000, reactive functional group: two hydroxy groups, 10 g, 250 μmol, moisture value: 1.1 mass%, GPC polydispersity: 1.11), the pressure was reduced to -0.1 MPaG or less using the vacuum pump, and the resultant was dried for 3 hours to obtain a dried particulate material having a moisture value of 0.01 mass% (step (A)).

After the step (A), the pressure was restored with nitrogen, and toluene (70 g, moisture value: 129 ppm) was charged using a glass syringe and a needle by holding the dried particulate material under a nitrogen atmosphere without contact with the outside air, followed by heating to 40°C and dissolution by stirring to obtain a solution (step (B)).

To the obtained solution, triethylamine (TEA, 58 mg, 1150 mmol) as a base catalyst and methanesulfonyl chloride (57 mg, 1,000 mmol) as an activator were added, and then the mixture was reacted at 40°C for 3 hours under nitrogen. Ethyl acetate (500 g) was added to the solution after the reaction for mixing at 40°C, hexane (500 g) was added thereto to carry out crystallization at 25°C, and then suction filtration was performed to obtain a crystal. An operation of adding ethyl acetate (500 g) to the obtained crystal for dissolution at 40°C and adding hexane (500 g) to carry out crystallization at 25°C was repeated to remove low molecular weight impurities, and then drying under a reduced pressure was performed to obtain a polyethylene glycol derivative represented by a formula (7) (step (C)).

The polyethylene glycol derivative obtained in the step (C) was subjected to 1H-NMR measurement at a number of times of integration of 256 times, and it was found that the activation rate was 85%. GPC measurement was performed, and it was found that the polydispersity was 1.10. Moisture measurement was performed, and it was found that the moisture value was 0.2 mass%.

[Chem. 6]

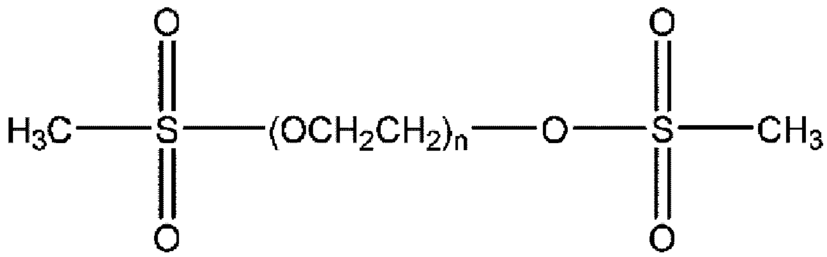

Formula (7)

(n = about 909)

<Example 2>

Into a 100 mL three-necked round-bottom flask equipped with a mechanical stirrer, an L-shaped tube connected to a vacuum pump, a check valve, and a three-way cock connected to a nitrogen line was charged a particulate material of a polyethylene glycol compound represented by a formula (8) (Mw: 43,000 Daltons, reactive functional group: one hydroxy group, 10 g, 233 μmol, moisture value: 0.1 mass%, GPC polydispersity: 1.04), the pressure was reduced to -0.1 MPaG or less using the vacuum pump, and the resultant was dried for 1 hour to obtain a dried particulate material having a moisture value of 0.02 mass% (step (A)).

After the step (A), the pressure was restored with nitrogen, and dichloromethane (70 g, moisture value: 42 ppm) was charged using a glass syringe and a needle to the dried particulate material under a nitrogen atmosphere without contact with the atmosphere, followed by dissolution by stirring at 25°C to obtain a solution (step (B)).

To the solution obtained in the step (B), disuccinimidyl carbonate (DSC, 918 mg, 3584 μmol) as an activator and pyridine (434 μL, 5233 μmol) as a base catalyst were added, and then the mixture was reacted at 27°C for 15 hours under nitrogen. Ethyl acetate (500 g) was added to the solution after the reaction for mixing at 40°C, hexane (500 g) was added thereto to carry out crystallization at 25°C, and then suction filtration was performed to obtain a crystal. An operation of adding ethyl acetate (500 g) to the obtained crystal for dissolution at 40°C and adding hexane (500 g) to carry out crystallization at 25°C was repeated to remove low molecular weight impurities, and then drying under a reduced pressure was performed to obtain an activated polyethylene glycol derivative represented by a formula (9) (step (C)).

The activated polyethylene glycol derivative obtained in the step (C) was subjected to 1H-NMR measurement at a number of times of integration of 256 times, and it was found that the activation rate was 98%. GPC measurement was performed, and it was found that the polydispersity was 1.04. Moisture measurement was performed, and it was found that the moisture value was 0.3 mass%.

[Chem. 7]

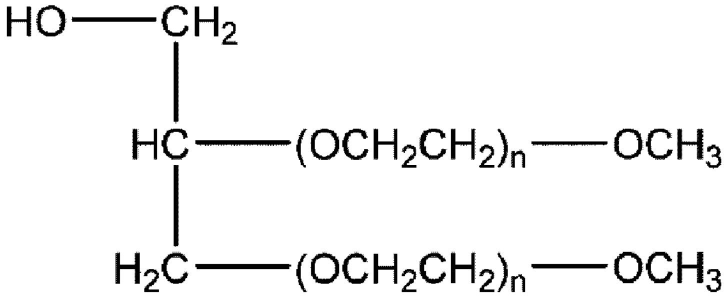

Formula (8)

(n = about 489)

[Chem. 8]

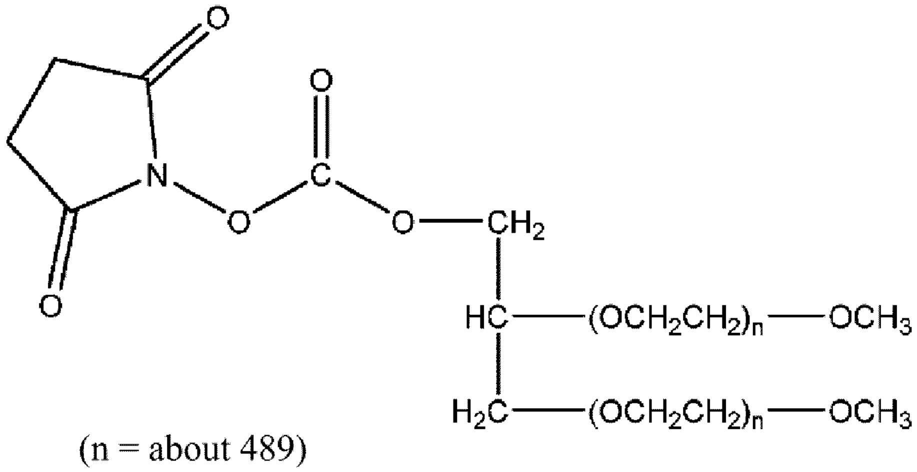

(9)

(n = about 489)

<Example 3>

Into a 100 mL three-necked round-bottom flask equipped with a mechanical stirrer, an L-shaped tube connected to a vacuum pump, a check valve, and a three-way cock connected to a nitrogen line were charged a particulate material of a polyethylene glycol compound represented by the formula (5) (Mw: 2,000 Daltons, reactive functional group: one hydroxy group, 5 g, 2.5 mmol, moisture value: 0.5 mass%, GPC polydispersity: 1.06) and sodium acetate (15 mg) as a base catalyst, the pressure was reduced to -0.1 MPaG or less using the vacuum pump, and the resultant was dried for 1 hour to obtain a dried particulate material having a moisture value of 0.08 mass% (step (A)).

After the step (A), the pressure was restored with nitrogen, and toluene (5 g, moisture value: 67 ppm) was charged using a glass syringe and a needle to the dried particulate material under a nitrogen atmosphere without contact with the atmosphere, followed by heating to 40°C and dissolution by stirring to obtain a solution (step (B)).

To the obtained solution, succinic anhydride (263 mg, 2.63 mmol) as an activator was added, and then the mixture was reacted at 70°C for 12 hours under nitrogen. Ethyl acetate (500 g) was added to the solution after the reaction for mixing at 40°C, hexane (500 g) was added thereto to carry out crystallization at 25°C, and then suction filtration was performed to obtain a crystal. An operation of adding ethyl acetate (500 g) to the obtained crystal for dissolution at 40°C and adding hexane (500 g) to carry out crystallization at 25°C was repeated to remove low molecular weight impurities, and then drying under a reduced pressure was performed to obtain an activated polyethylene glycol derivative represented by a formula (10) (step (C)).

The activated polyethylene glycol derivative obtained in the step (C) was subjected to 1H-NMR measurement at a number of times of integration of 64 times, and it was found that the activation rate was 91%. GPC measurement was performed, and it was found that the polydispersity was 1.06. Moisture measurement was performed, and it was found that the moisture value was 0.3 mass%.

[Chem. 9]

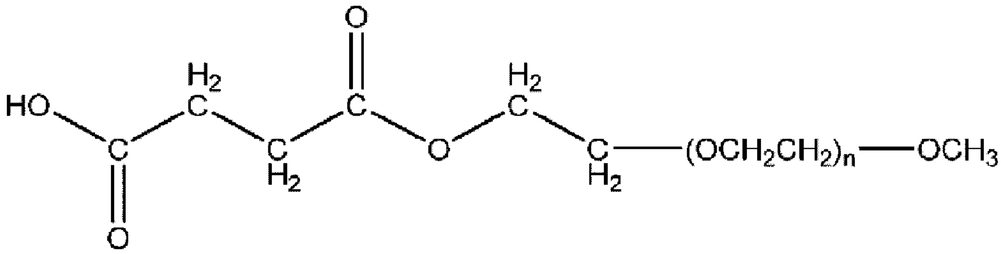

(1 0)

(n = about 45)

<Example 4>

Into a 100 mL three-necked round-bottom flask equipped with a mechanical stirrer, an L-shaped tube connected to a vacuum pump, a check valve, and a three-way cock connected to a nitrogen line were charged a particulate material of a polyethylene glycol compound represented by the formula (11) (Mw: 10,000 Daltons, reactive functional group: four amino groups, 5 g, 500 μmol, moisture value: 0.5 mass%, GPC polydispersity: 1.06) and sodium acetate (15 mg) as a base catalyst, the pressure was reduced to -0.1 MPaG or less using the vacuum pump, and the resultant was dried for 2 hours to obtain a dried particulate material having a moisture value of 0.07 mass% (step (A)).

After the step (A), the pressure was restored with nitrogen, and toluene (5 g, moisture value: 92 ppm) was charged using a glass syringe and a needle to the dried particulate material under a nitrogen atmosphere without contact with the atmosphere, followed by heating to 40°C and dissolution by stirring to obtain a solution (step (B)).

To the solution in the step (B), succinic anhydride (210 mg, 525 μmol) as an activator was added, and then the mixture was reacted at 70°C for 12 hours under nitrogen. Ethyl acetate (500 g) was added to the solution after the reaction for mixing at 40°C, hexane (500 g) was added thereto to carry out crystallization at 25°C, and then suction filtration was performed to obtain a crystal. An operation of adding ethyl acetate (500 g) to the obtained crystal for dissolution at 40°C and adding hexane (500 g) to carry out crystallization at 25°C was repeated to remove low molecular weight impurities, and then drying under a reduced pressure was performed to obtain an activated polyethylene glycol derivative represented by a formula (12) (step (C)).

The activated polyethylene glycol derivative obtained in the step (C) was subjected to 1H-NMR measurement at a number of times of integration of 128 times, and it was found that the activation rate was 95%. GPC measurement was performed, and it was found that the polydispersity was 1.05. Moisture measurement was performed, and it was found that the moisture value was 0.5 mass%.

[Chem. 10]

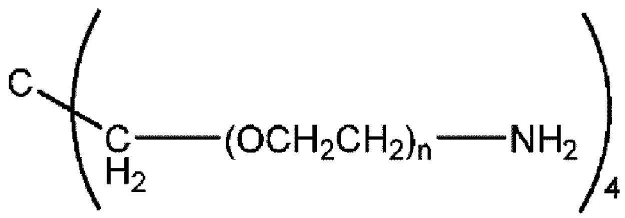

(1 1)

(n = about 57)

[Chem. 11]

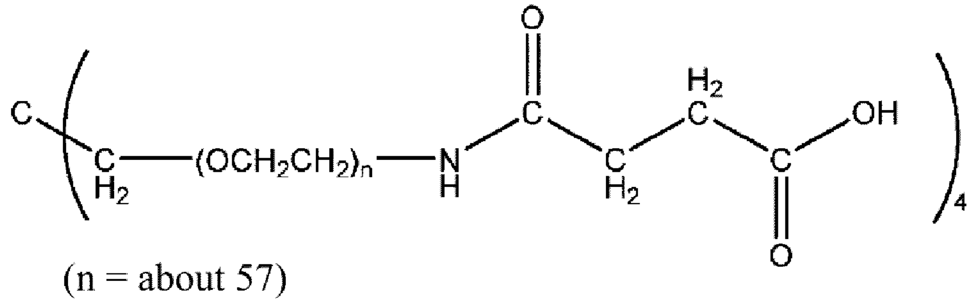

(n = about 57)

(12)

<Example 5>

Into a 20 mL screw-top glass bottle was charged a particulate material of a polyethylene glycol compound represented by the formula (5) (Mw: 2,000 Daltons, reactive functional group: one hydroxy group, 1 g, 500 μmol, moisture value: 0.5 mass%, GPC polydispersity: 1.03), and the screw-top glass bottle was placed into a glove box, followed by drying by stirring for 1 hour with a spatula while circulating dried nitrogen, to obtain a dried particulate material having a moisture value of 0.08 mass% (step (A)).

After the step (A), a stirring bar was placed into the screw-top glass bottle in the glove box, toluene (2 g, moisture value: 62 ppm) and chloroform (18 g, moisture value: 68 ppm) were charged thereto, the lid was closed, then the screw-top glass bottle was taken out of the glove box, and the mixture was dissolved by stirring with a magnetic stirrer at 25°C to obtain a solution (step (B)).

To the obtained solution, phthalimide (129 mg, 875 μmol), triphenylphosphine (TPP, 230 mg, 875 μmol), and diisopropyl azodicarboxylate (DIAD, 152 mg, 750 mol) were added, and then the mixture was reacted at 25°C for 2 hours and 30 minutes under nitrogen. Ethyl acetate (100 g) was added to the solution after the reaction for mixing at 40°C, hexane (100 g) was added thereto to carry out crystallization at 25°C, and then suction filtration was performed to obtain a crystal. An operation of adding ethyl acetate (100 g) to the obtained crystal for dissolution at 40°C and adding hexane (100 g) to carry out crystallization at 25°C was repeated to remove low molecular weight impurities, and then drying under a reduced pressure was performed to obtain an activated polyethylene glycol derivative represented by a formula (13) (step (C)).

The activated polyethylene glycol derivative obtained in the step (C) was subjected to 1H-NMR measurement at a number of times of integration of 64 times, and it was found that the activation rate was 88%. GPC measurement was performed, and it was found that the polydispersity was 1.03. Moisture measurement was performed, and it was found that the moisture value was 0.5 mass%.

[Chem. 12]

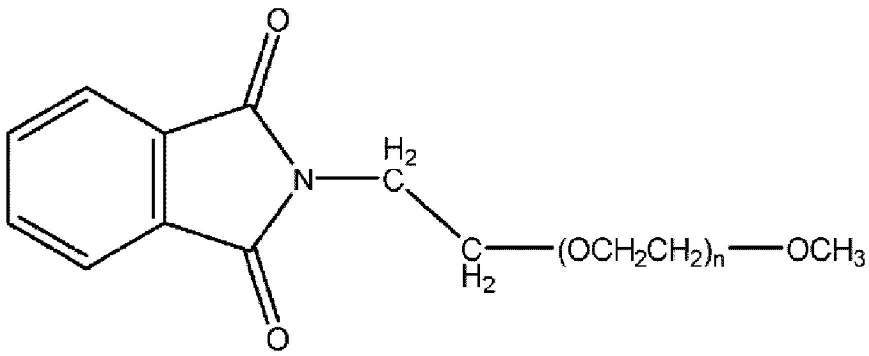

(n = about 45)

(13)

<Example 6>

Into a 100 mL three-necked round-bottom flask equipped with a mechanical stirrer, an L-shaped tube connected to a vacuum pump, a check valve, and a three-way cock connected to a nitrogen line was charged a particulate material of a polyethylene glycol compound represented by the formula (6) (Mw: 40,000 Daltons, reactive functional group: two hydroxy groups, 10 g, 250 μmol, moisture value: 1.1 mass%, GPC polydispersity: 1.11), the pressure was reduced to -0.1 MPaG or less using the vacuum pump, and the resultant was dried for 1 hour to obtain a dried particulate material having a moisture

value of 0.03 mass% (step (A)).

After the step (A), the pressure was restored with nitrogen, and dichloromethane (70 g, moisture value: 51 ppm) was charged using a glass syringe and a needle to the dried particulate material under a nitrogen atmosphere without contact with the atmosphere, followed by dissolution by stirring at 25°C to obtain a solution (step (B)).

To the solution obtained in the step (B), disuccinimidyl carbonate (DSC, 643 mg, 2510 μmol) as an activator and pyridine (303 μL, 3750 μmol) as a base catalyst were added, and then the mixture was reacted at 25°C for 16 hours under nitrogen. Ethyl acetate (500 g) was added to the solution after the reaction for mixing at 40°C, hexane (500 g) was added thereto to carry out crystallization at 25°C, and then suction filtration was performed to obtain a crystal. An operation of adding ethyl acetate (500 g) to the obtained crystal for dissolution at 40°C and adding hexane (500 g) to carry out crystallization at 25°C was repeated to remove low molecular weight impurities, and then drying under a reduced pressure was performed to obtain an activated polyethylene glycol derivative represented by a formula (14) (step (C)).

The activated polyethylene glycol derivative obtained in the step (C) was subjected to 1H-NMR measurement at a number of times of integration of 256 times, and it was found that the activation rate was 100%. GPC measurement was performed, and it was found that the polydispersity was 1.11. Moisture measurement was performed, and it was found that the moisture value was 0.3 mass%.

[Chem. 13]

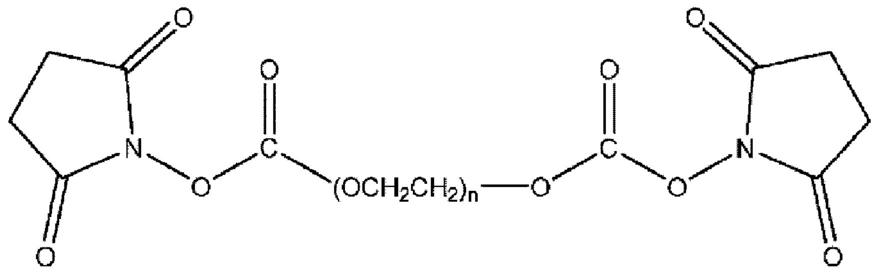

(n = about 909)

(14)

<Example 7>

Into a 100 mL three-necked round-bottom flask equipped with a mechanical stirrer, an L-shaped tube connected to a vacuum pump, a check valve, and a three-way cock connected to a nitrogen line was charged a particulate material of a polyethylene glycol compound represented by a formula (15) (Mw: 80,000 Daltons, reactive functional group: one hydroxy group, 2 g, 25 μmol, moisture value: 1.0 mass%, GPC polydispersity: 1.06), the pressure was reduced to -0.1 MPaG or less using the vacuum pump, and the resultant was dried for 3 hours to obtain a dried particulate material having a moisture value of 0.08 mass% (step (A)).

After the step (A), the pressure was restored with nitrogen, and dichloromethane (10 g, moisture value: 106 ppm) was charged using a glass syringe and a needle to the dried particulate material under a nitrogen atmosphere without contact with the atmosphere, followed by dissolution by stirring at 25°C to obtain a solution (step (B)).

To the solution obtained in the step (B), disuccinimidyl carbonate (DSC, 96 mg, 375 μmol) as an activator and pyridine (46 μL, 569 μmol) as a base catalyst were added, and then the mixture was reacted at 25°C for hours under nitrogen. Ethyl acetate (500 g) was added to the solution after the reaction for mixing at 40°C, hexane (500 g) was added thereto to carry out crystallization at 25°C, and then suction filtration was performed to obtain a crystal. An operation of adding ethyl acetate (500 g) to the obtained crystal for dissolution at 40°C and adding hexane (500 g) to carry out crystallization at 25°C was repeated to remove low molecular weight impurities, and then drying under a reduced pressure was performed to obtain an activated polyethylene glycol derivative represented by a formula (16) (step (C)).

The activated polyethylene glycol derivative obtained in the step (C) was subjected to 1H-NMR measurement at a number of times of integration of 256 times, and it was found that the activation rate was 100%. GPC measurement was performed, and it was found that the polydispersity was 1.06. Moisture measurement was performed, and it was found that the moisture value had a mass of 0.3%.

[Chem. 14]

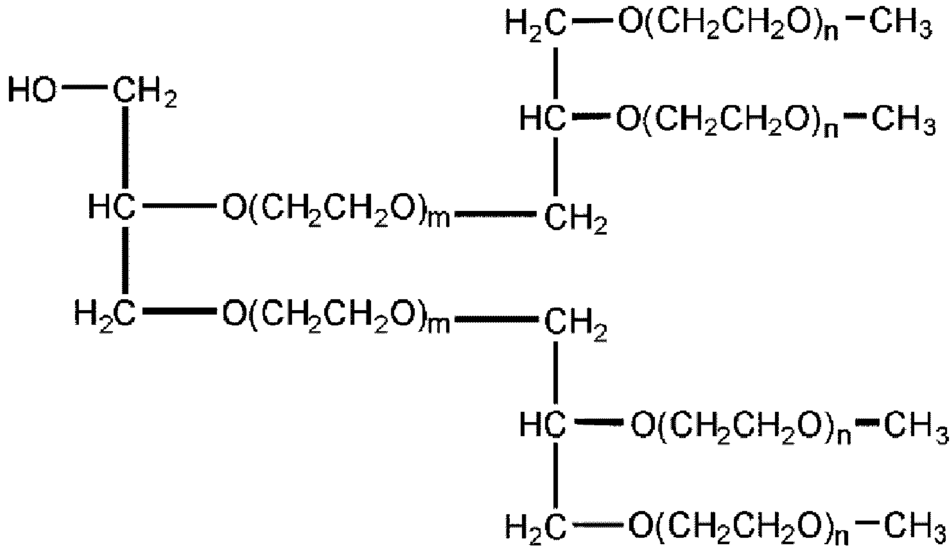

(n = about 114, n = about 398)

(15)

[Chem. 15]

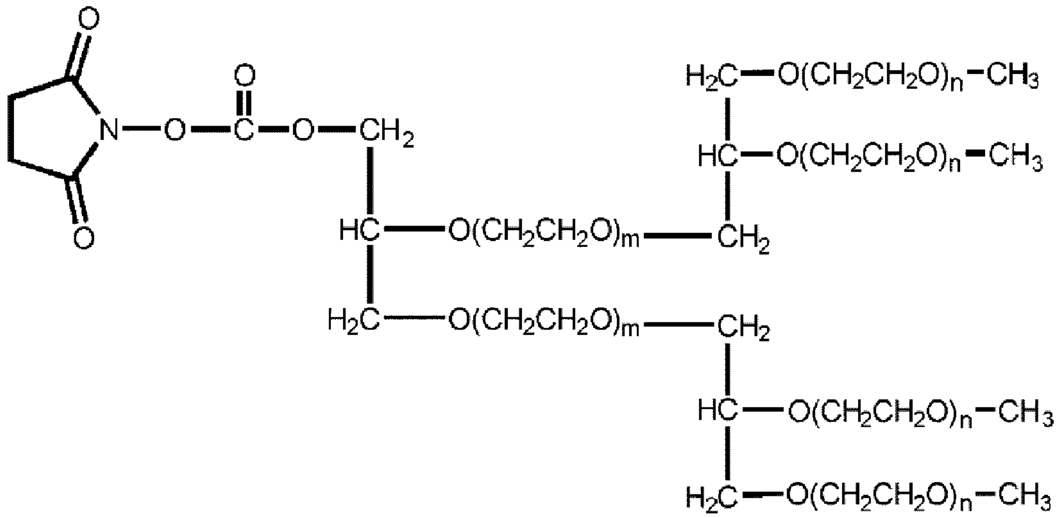

(n = about 114, n = about 398)

(16)

<Example 8>

Into a 100 mL three-necked round-bottom flask equipped with a mechanical stirrer, an L-shaped tube connected to a vacuum pump, a check valve, and a three-way cock connected to a nitrogen line was charged a particulate material of a polyethylene glycol compound represented by a formula (17) (Mw: 40,000 Daltons, reactive functional group: eight hydroxy groups, 2 g, 50 μmol, moisture value: 0.8 mass%, GPC polydispersity: 1.07), the pressure was reduced to -0.1 MPaG or less using the vacuum pump, and the resultant was dried for 3 hours to obtain a dried particulate material having a moisture value of 0.09 mass% (step (A)).

After the step (A), the pressure was restored with nitrogen, and dichloromethane (10 g, moisture value: 104 ppm) was charged using a glass syringe and a needle to the dried particulate material under a nitrogen atmosphere without

contact with the atmosphere, followed by dissolution by stirring at 25°C to obtain a solution (step (B)).

To the solution obtained in the step (B), disuccinimidyl carbonate (DSC, 206 mg, 804 μmol) as an activator and pyridine (97 μL, 1199 μmol) as a base catalyst were added, and then the mixture was reacted at 25°C for hours under nitrogen. Ethyl acetate (500 g) was added to the solution after the reaction for mixing at 40°C, hexane (500 g) was added thereto to carry out crystallization at 25°C, and then suction filtration was performed to obtain a crystal. An operation of adding ethyl acetate (500 g) to the obtained crystal for dissolution at 40°C and adding hexane (500 g) to carry out crystallization at 25°C was repeated to remove low molecular weight impurities, and then drying under a reduced pressure was performed to obtain an activated polyethylene glycol derivative represented by a formula (18) (step (C)).

The activated polyethylene glycol derivative obtained in the step (C) was subjected to 1H-NMR measurement at a number of times of integration of 256 times, and it was found that the activation rate was 99%. GPC measurement was performed, and it was found that the polydispersity was 1.07. Moisture measurement was performed, and it was found that the moisture value had a mass of 0.3%.

[Chem. 16]

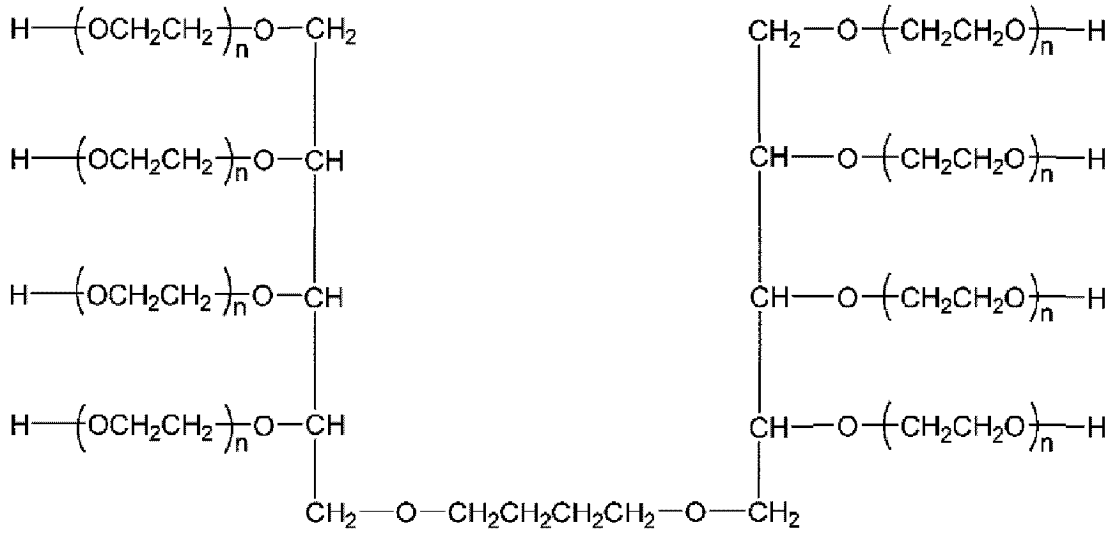

(17)

(n = about 56)

[Chem. 17]

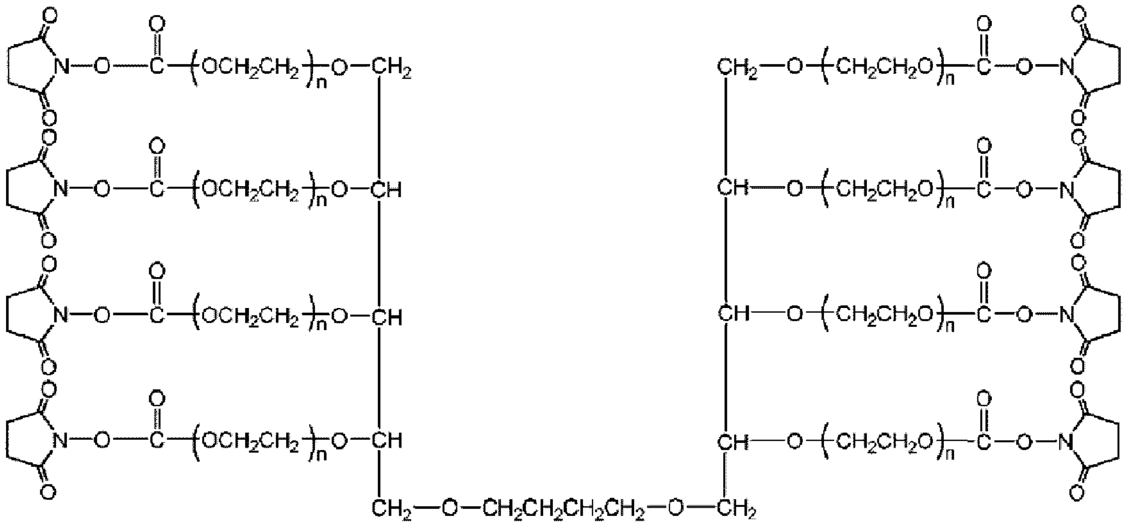

(n = about 56)

(18)

<Example 9>

Into a 100 mL three-necked round-bottom flask equipped with a mechanical stirrer, an L-shaped tube connected to a vacuum pump, a check valve, and a three-way cock connected to a nitrogen line were charged a particulate material of a polyethylene glycol compound represented by the formula (5) (Mw: 2,000 Daltons, reactive functional group: one hydroxy group, 10 g, 5.0 mmol, moisture value: 0.5 mass%, GPC polydispersity: 1.03) and sodium acetate (3 mg) as a base catalyst, the pressure was reduced to -0.1 MPaG or less using the vacuum pump, and the resultant was dried for 3 hours to obtain a dried particulate material having a moisture value of 0.03 mass% (step (A)).

After the step (A), the pressure was restored with nitrogen, and 4-methyltetrahydropyran (MTHP, 10 g, moisture value: 94 ppm) was charged using a glass syringe and a needle to the dried particulate material under a nitrogen atmosphere without contact with the atmosphere, followed by heating to 40°C and dissolution by stirring to obtain a solution (step (B)).

To the obtained solution, succinic anhydride (528 mg, 5.28 mmol) as an activator was added, and then the mixture was reacted at 70°C for 12 hours under nitrogen. Ethyl acetate (500 g) was added to the solution after the reaction for mixing at 40°C, hexane (500 g) was added thereto to carry out crystallization at 25°C, and then suction filtration was performed to obtain a crystal. An operation of adding ethyl acetate (500 g) to the obtained crystal for dissolution at 40°C and adding hexane (500 g) to carry out crystallization at 25°C was repeated to remove low molecular weight impurities, and then drying under a reduced pressure was performed to obtain an activated polyethylene glycol derivative represented by the formula (10) (step (C)).

The activated polyethylene glycol derivative obtained in the step (C) was subjected to 1H-NMR measurement at a number of times of integration of 64 times, and it was found that the activation rate was 85%. GPC measurement was performed, and it was found that the polydispersity was 1.03. Moisture measurement was performed, and it was found that the moisture value was 0.4 mass%.

<Example 10>

Into a 100 mL three-necked round-bottom flask equipped with a mechanical stirrer, an L-shaped tube connected to a vacuum pump, a check valve, and a three-way cock connected to a nitrogen line was charged a particulate material of a polyethylene glycol compound represented by a formula (19) (Mw: 5,000 Daltons, reactive functional group: one carbonyl group, 2 g, 400 μmol, moisture value: 0.9 mass%, GPC polydispersity: 1.03), the pressure was reduced to -0.1 MPaG or less using the vacuum pump, and the resultant was dried for 3 hours to obtain a dried particulate material having a moisture value of 0.02 mass% (step (A)).

After the step (A), the pressure was restored with nitrogen, and toluene (10 g, moisture value: 83 ppm) was charged using a glass syringe and a needle to the dried particulate material under a nitrogen atmosphere without contact with the atmosphere, followed by heating to 40°C and dissolution by stirring to obtain a solution (step (B)).

To the obtained solution, N,N'-dicyclohexylcarbodiimide (DCC, 165 mg, 800 μmol) as an activator and N-hydroxysuccinimide (NHS, 101 mg, 878 μmol) were added, and then the mixture was reacted at 40°C for 3 hours under nitrogen. Ethyl acetate (500 g) was added to the solution after the reaction for mixing at 40°C, hexane (500 g) was added to a filtrate obtained by suction filtration to carry out crystallization at 25°C, and then suction filtration was performed to obtain a crystal. An operation of adding ethyl acetate (500 g) to the obtained crystal for dissolution at 40°C and adding hexane (500 g) to carry out crystallization at 25°C was repeated to remove low molecular weight impurities, and then drying under a reduced pressure was performed to obtain an activated polyethylene glycol derivative represented by a formula (20) (step (C)).

The activated polyethylene glycol derivative obtained in the step (C) was subjected to 1H-NMR measurement at a number of times of integration of 128 times, and it was found that the activation rate was 99%. GPC measurement was performed, and it was found that the polydispersity was 1.03. Moisture measurement was performed, and it was found that the moisture value was 0.2 mass%.

[Chem. 18]

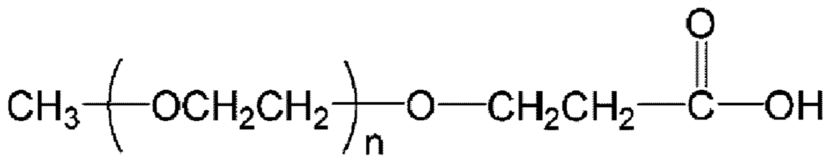

(19)

(n = about 114)

[Chem. 19]

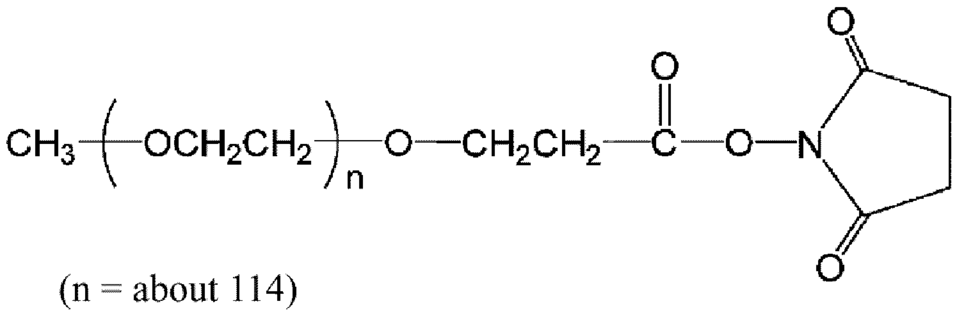

(n = about 114)

(20)

<Example 11>

Into a 100 mL three-necked round-bottom flask equipped with a mechanical stirrer, an L-shaped tube connected to a vacuum pump, a check valve, and a three-way cock connected to a nitrogen line was charged a particulate material of a polyethylene glycol compound represented by the formula (5) (Mw: 2,000 Daltons, reactive functional group: one hydroxy group, 10 g, 5.0 mmol, moisture value: 0.5 mass%, GPC polydispersity: 1.03), the pressure was reduced to -0.1 MPaG or less using the vacuum pump, and the resultant was dried for 3 hours to obtain a dried particulate material having a moisture value of 0.01 mass% (step (A)).

After the step (A), the pressure was restored with nitrogen, and toluene (11 g, moisture value: 93 ppm) was charged using a glass syringe and a needle to the dried particulate material under a nitrogen atmosphere without contact with the atmosphere, followed by heating to 40°C and dissolution by stirring to obtain a solution (step (B)).

To the obtained solution, p-nitrophenyl chloroformate (1.11 g, 5.5 mmol) as an activator and triethylamine (TEA, 837 μL, 6.0 μmol) as a base catalyst were added, and then the mixture was reacted at 60°C for 2 hours under nitrogen. Ethyl acetate (500 g) was added to the solution after the reaction for mixing at 40°C, hexane (500 g) was added thereto to carry out crystallization at 25°C, and then suction filtration was performed to obtain a crystal. An operation of adding ethyl acetate (500 g) to the obtained crystal for dissolution at 40°C and adding hexane (500 g) to carry out crystallization at 25°C was repeated to remove low molecular weight impurities, and then drying under a reduced pressure was performed to obtain an activated polyethylene glycol derivative represented by a formula (21) (step (C)).

The activated polyethylene glycol derivative obtained in the step (C) was subjected to 1H-NMR measurement at a number of times of integration of 64 times, and it was found that the activation rate was 91%. GPC measurement was performed, and it was found that the polydispersity was 1.03. Moisture measurement was performed, and it was found that the moisture value was 0.3 mass%.

[Chem. 20]

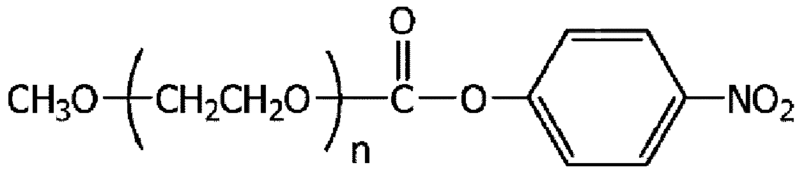

(21)

(n = about 45)

In order to check the influence on the reaction in the case where the particulate material of the polyethylene glycol compound absorbs moisture, the following studies were performed.

<Comparative Example 1>

Into a 300 mL four-necked round-bottom flask equipped with a mechanical stirrer, a Dimroth cooling tube, a thermometer, and a nitrogen injection tube was charged a particulate material of a polyethylene glycol compound represented by the formula (6) (Mw: 40,000 Daltons, reactive functional group: two hydroxy groups, 10 g, 250 μmol, moisture value: 1.1 mass%, GPC polydispersity: 1.11), toluene (70 g, moisture value: 41 ppm) was added thereto, and then the mixture was heated to 40°C in a water bath and dissolved by stirring to obtain a solution.

To the obtained solution, triethylamine (TEA, 58 mg, 1150 μmol) and methanesulfonyl chloride (MsCl, 57 mg, 1,000 μmol) were added, and then the mixture was reacted at 40°C for 3 hours under nitrogen. Ethyl acetate (500 g) was added to the solution after the reaction for mixing at 40°C, hexane (500 g) was added thereto to carry out crystallization at 25°C, and then suction filtration was performed to obtain a crystal. An operation of adding ethyl acetate (500 g) to the obtained crystal for dissolution at 40°C and adding hexane (500 g) to carry out crystallization at 25°C was repeated to remove low molecular weight impurities, and then drying under a reduced pressure was performed to obtain an activated polyethylene glycol derivative represented by the formula (7).

The obtained activated polyethylene glycol derivative was subjected to 1H-NMR measurement at a number of times of integration of 256 times, and it was found that the activation rate was very low at 25%. GPC measurement was performed, and it was found that the polydispersity was 1.11. Moisture measurement was performed, and it was found that the moisture value was 0.2 mass%.

<Comparative Example 2>

Into a 100 mL three-necked round-bottom flask equipped with a mechanical stirrer, a Dimroth cooling tube, a thermometer, and a nitrogen injection tube was charged a particulate material of a polyethylene glycol compound represented by the formula (6) (Mw: 40,000 Daltons, reactive functional group: two hydroxy groups, 10 g, 250 μmol, moisture value: 0.8 mass%, GPC polydispersity: 1.06), dichloromethane (72 g, moisture value: 104 ppm) was added thereto, and then the mixture was dissolved by stirring at 25°C to obtain a solution.

To the obtained solution, disuccinimidyl carbonate (DSC, 643 mg, 2510 μmol) as an activator and pyridine (303 μL, 3750 μmol) as a base catalyst were added, and then the mixture was reacted at 25°C for 18 hours under nitrogen. Ethyl acetate (500 g) was added to the solution after the reaction for mixing at 40°C, hexane (500 g) was added thereto to carry out crystallization at 25°C, and then suction filtration was performed to obtain a crystal. An operation of adding ethyl acetate (500 g) to the obtained crystal for dissolution at 40°C and adding hexane (500 g) to carry out crystallization at 25°C was repeated to remove low molecular weight impurities, and then drying under a reduced pressure was performed to obtain an activated polyethylene glycol derivative represented by the formula (14).

The obtained activated polyethylene glycol derivative was subjected to 1H-NMR measurement at a number of times of integration of 256 times, and it was found that the activation rate was very low at 61%. GPC measurement was performed, and it was found that the polydispersity was 1.06. Moisture measurement was performed, and it was found that the moisture value was 0.3 mass%.

Table 3 shows the results for Example 1 and Comparative Example 1, and Example 6 and Comparative Example 2. As shown in Table 3, it is found that, in Comparative Examples 1 and 2, the activation rate of the activated polyethylene glycol derivative significantly decreases.

[Table 3]

| | Particulate material of polyethylene glycol derivative | | | Drying | | | Reaction | | |
|---|---|---|---|---|---|---|---|---|---|
| | Structural formula | Number average molecular weight (Daltons) | Reactive functional group | Drying method | Reaction solvent | Increase in GPC polydispersity | Activator | Reaction accelerator | Activation rate (%) |
| Example 1 | (6) | 40,000 | Two hydroxy groups | Reduced pressure | Toluene | No | MsCl | TEA | 85 |
| Comparative Example 1 | (6) | 40,000 | Two hydroxy groups | No | Toluene | No | MsCl | TEA | 25 |
| Example 6 | (6) | 40,000 | Two hydroxy groups | Reduced pressure | Dichloromethane | No | DSC | Pyridine | 100 |
| Comparative Example 2 | (6) | 40,000 | Two hydroxy groups | No | Dichloromethane | No | DSC | Pyridine | 61 |

Table 4 shows the results for Examples 1 to 11. As shown in Table 4, it is found that, according to the present invention, a polyethylene glycol derivative having a high activation purity can be produced without increasing the polydispersity even under the reaction conditions.

**[Table 4]**

| | Particulate material of polyethylene glycol derivative | | | Drying | | | Reaction | | |
|---|---|---|---|---|---|---|---|---|---|
| | Structural formula | Number average molecular weight (Daltons) | Reactive functional group | Drying method | Reaction solvent | Increase in GPC polydispersity | Activator | Reaction accelerator | Activation rate (%) |
| Example 1 | (6) | 40,000 | Two hydroxy groups | Reduced pressure | Toluene | No | MsCl | TEA | 85 |
| Example 2 | (8) | 43,000 | One hydroxy group | Reduced pressure | Dichloromethane | No | DSC | Pvridine | 98 |
| Example 3 | (5) | 2,000 | One hydroxy group | Reduced pressure | Toluene | No | Succinic anhydride | Sodium acetate | 91 |
| Example 4 | (11) | 10,000 | Four amino groups | Reduced pressure | Toluene | No | Succinic anhydride | Sodium acetate | 95 |
| Example 5 | (5) | 2,000 | One hydroxy group | Dry gas circulation (nitrogen) | Toluene and chloroform | No | Phthalimide, TPP, and DIAD | - | 88 |
| Example 6 | (6) | 40,000 | Two hydroxy groups | Reduced pressure | Dichloromethane | No | DSC | Pvridine | 100 |
| Example 7 | (15) | 80,000 | One hydroxy group | Reduced pressure | Dichloromethane | No | DSC | Pyridine | 100 |
| Example 8 | (17) | 40,000 | Eight hydroxy groups | Reduced pressure | Dichloromethane | No | DSC | Pyridine | 99 |
| Example 9 | (5) | 2,000 | One hydroxy group | Reduced pressure | MTHP | No | Succinic anhvdride | Sodium acetate | 85 |
| Example 10 | (19) | 5,000 | One carbonyl group | Reduced pressure | Toluene | No | DCC and NHS | - | 99 |
| Example 11 | (5) | 2,000 | One hydroxy group | Reduced pressure | Toluene | No | p-Nitrophenyl chloroformate | TEA | 91 |

INDUSTRIAL APPLICABILITY

According to the present invention, it is possible to obtain an effect of increasing the activation purity, preventing an increase in polydispersity due to an increase in thermal history, and producing an industrially feasible activated polyethylene glycol derivative.

Although the present invention has been described in detail and with reference to specific embodiments, it will be apparent to those skilled in the art that various changes and modifications can be made without departing from the spirit and scope of the present invention.

The present application is based on a Japanese patent application (Japanese Patent Application No. 2023-051234) filed on March 28, 2023, the contents of which are incorporated herein by reference.

## Claims

1. A method for producing an activated polyethylene glycol derivative having, at a terminal, a functional group reactive with a bio-related substance or a precursor of the functional group, the method comprising the following step (A), step (B), and step (C):

    step (A): a step of drying a particulate material of a polyethylene glycol compound having, at a terminal, one or more groups selected from the group consisting of a carboxy group, a mercapto group, a hydroxy group, and an amino group under an atmosphere of 0°C or higher and 50°C or lower to obtain a dried particulate material having a moisture value of 0.10 mass% or less;
    step (B): a step of adding an organic solvent containing no more than 200 ppm of water to the dried particulate material obtained in the step (A) to dissolve the dried particulate material in the organic solvent to obtain a solution; and
    step (C): a step of reacting the polyethylene glycol compound contained in the solution obtained in the step (B) with an activator to obtain an activated polyethylene glycol derivative.

2. The method for producing an activated polyethylene glycol derivative according to claim 1, wherein the organic solvent is an aprotic solvent.

3. The method for producing an activated polyethylene glycol derivative according to claim 1 or 2, wherein the particulate material in the step (A) has an average particle diameter of 0.1 μm or more and 10 mm or less.

4. The method for producing an activated polyethylene glycol derivative according to claim 1 or 2, wherein the polyethylene glycol compound in the step (A) has a number average molecular weight of 2,000 Daltons or more and 80,000 Daltons or less.

5. The method for producing a polyethylene glycol derivative according to claim 1 or 2, wherein the activated polyethylene glycol derivative is represented by the following formula (1):

$$\text{PEG-X} \quad \cdots (1)$$

    (in the formula (1),

    PEG represents a polyethylene glycol moiety having a linear structure or a branched structure, and
    X represents the functional group reactive with the bio-related substance or the precursor of the functional group).

6. The method for producing a polyethylene glycol derivative according to claim 1 or 2, wherein the activator is one or more compounds selected from the group consisting of disuccinimidyl carbonate, p-nitrophenyl chloroformate, di(1-benzotriazolyl) carbonate, trichlorophenyl chloroformate, p-nitrophenyl succinimidyl carbonate, p-nitrophenyl **1-**benzotriazolyl carbonate, pentafluorophenyl chloroformate, **1,1'-carbonyldiimidazole,** succinic anhydride, glutaric anhydride, halogenated methanesulfonyl, halogenated trifluoromethanesulfonyl, halogenated p-toluenesulfonyl, an alkyl 6-halogenated hexanoate, an azodicarboxylate, N,N'-dicyclohexylcarbodiimide, and N-hydroxysuccinimide.

FIG. 1

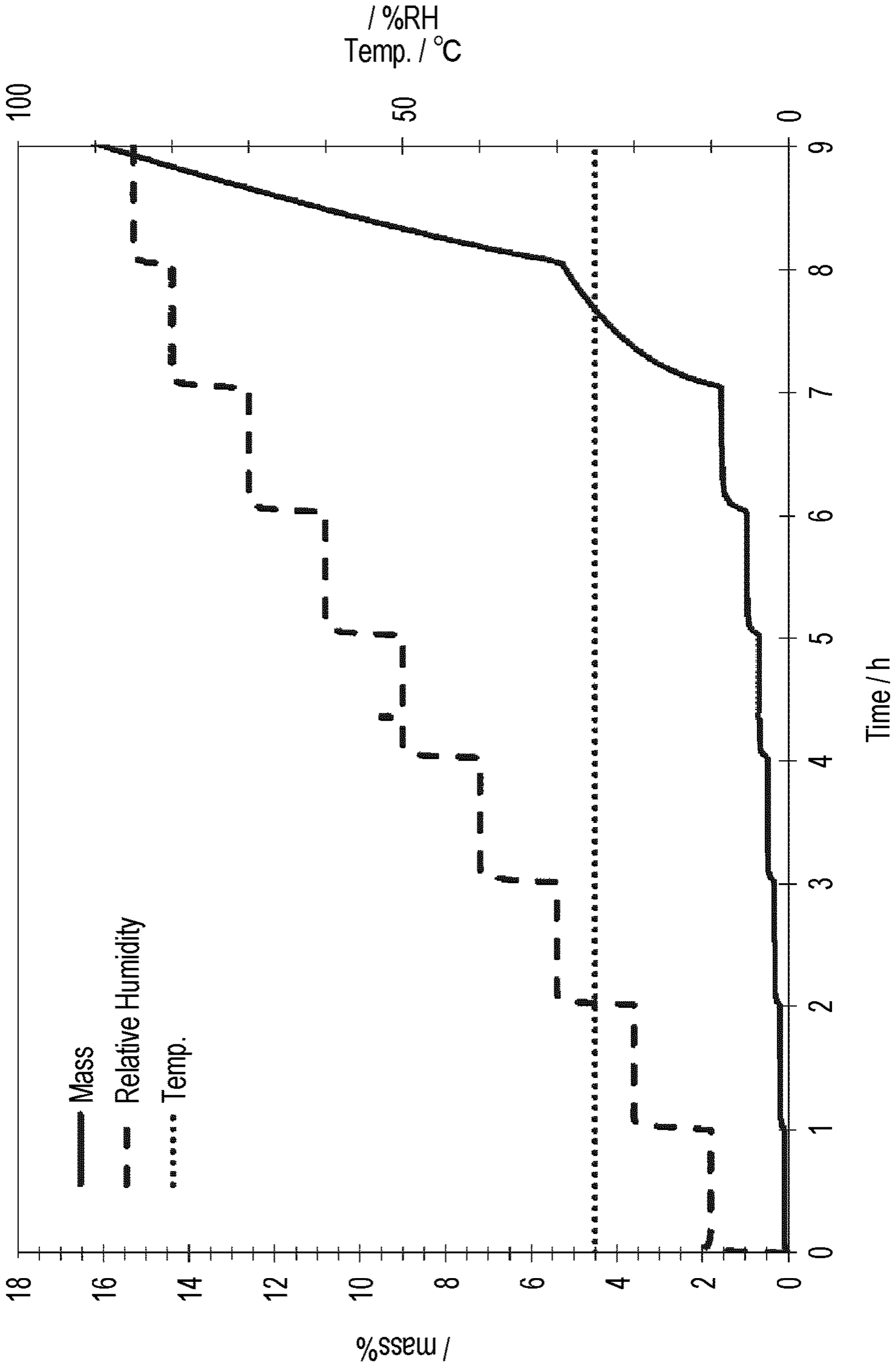

## INTERNATIONAL SEARCH REPORT

International application No.
**PCT/JP2024/010703**

**A. CLASSIFICATION OF SUBJECT MATTER**

***C08G 65/329***(2006.01)i
FI: C08G65/329

According to International Patent Classification (IPC) or to both national classification and IPC

**B. FIELDS SEARCHED**

Minimum documentation searched (classification system followed by classification symbols)

C08G65/00-65/48

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 7-112974 A (NOF CORPORATION) 02 May 1995 (1995-05-02) claims, paragraphs [0010]-[0012], examples | 1-6<br>1-6 |
| X<br>Y | JP 2004-525212 A (NEKTAR THERAPEUTICS AL CORP.) 19 August 2004 (2004-08-19) claims, paragraph [0032], examples | 1-6<br>1-6 |
| X<br>Y | JP 2019-167532 A (NOF CORPORATON) 03 October 2019 (2019-10-03) claims, examples | 1-6<br>1-6 |
| Y | JP 2017-95565 A (NOF CORPORATON) 01 June 2017 (2017-06-01) claims, paragraphs [0003], [0004], [0012], examples | 1-6 |
| Y | WO 2020/203583 A1 (NOF CORPORATON) 08 October 2020 (2020-10-08) claims, examples | 1-6 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 May 2024** | **11 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (July 2022)

**INTERNATIONAL SEARCH REPORT**

International application No.
**PCT/JP2024/010703**

**C. DOCUMENTS CONSIDERED TO BE RELEVANT**

| Category* | Citation of document, with indication, where appropriate, of the relevant passages | Relevant to claim No. |
|---|---|---|
| P, A | WO 2023/176809 A1 (NOF CORPORATON) 21 September 2023 (2023-09-21) claims, paragraph [0015] | 1-6 |

Form PCT/ISA/210 (second sheet) (July 2022)

**INTERNATIONAL SEARCH REPORT**
**Information on patent family members**

International application No.
**PCT/JP2024/010703**

| Patent document cited in search report | Publication date (day/month/year) | Patent family member(s) | Publication date (day/month/year) |
|---|---|---|---|
| JP 7-112974 A | 02 May 1995 | (Family: none) | |
| JP 2004-525212 A | 19 August 2004 | US 2002/0082345 A1<br>claims, paragraph [0037], examples<br>KR 10-0807141 B1 | |
| JP 2019-167532 A | 03 October 2019 | US 2021/0009757 A1<br>claims, examples<br>CN 111868021 A<br>KR 10-2020-0132878 A | |
| JP 2017-95565 A | 01 June 2017 | US 2019/0055353 A1<br>claims, paragraphs [0003], [0004], [0014], examples<br>CN 108350167 A | |
| WO 2020/203583 A1 | 08 October 2020 | US 2022/0185957 A1<br>claims, examples<br>CN 113631631 A<br>KR 10-2021-0148151 A | |
| WO 2023/176809 A1 | 21 September 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013227543 A
- JP 2023051234 A